# EUROPÄISCHE PATENTSCHRIFT

(11) **EP 0 613 679 B1**
(45) Veröffentlichungstag und Bekanntmachung des Hinweises auf die Patenterteilung: **13.11.1996**
(21) Anmeldenummer: 94103101.5
(22) Anmeldetag: 02.03.1994
(51) Int. Cl.: A61K 7/40, A61K 7/48

(54) **Organosiloxane enthaltende, kosmetische Zusammensetzungen**
Cosmetic compositions containing organosiloxanes
Compositions cosmétiques contenant des organosiloxanes

(30) Priorität: 02.03.1993 DE 4306481
(43) Veröffentlichungstag der Anmeldung: 07.09.1994
(73) Patentinhaber: Wacker-Chemie GmbH, D-81737 München (DE)
(72) Erfinder: Sejpka, Johann, Dr., D-84533 Marktl (DE); Huber, Annemarie, D-84533 Haiming (DE)

(56) Entgegenhaltungen:
- EP-A- 0 268 950
- EP-A- 0 281 394
- DE-A- 2 109 051
- US-A- 4 423 041
- US-A- 4 425 364

## Beschreibung

Die Erfindung betrifft kosmetische Zusammensetzungen, welche mindestens ein wachsartiges und mindestens ein flüchtiges Organosiloxan enthalten, sowie ein Verfahren zu deren Herstellung.

Organosiloxane enthaltende, kosmetische Zubereitungen zur Behandlung von Haaren, Nägeln und Haut sind bereits vielfach bekannt. Beispielsweise werden in EP-B-0 133 964 (Revlon, Inc.; ausgegeben am 4. Juli 1990) wasserfreie homogene kosmetische Mittel beschrieben, die Dimethylpolysiloxan und Organosilan oder Organosiloxan mit mindestens einer -SiR¹R²O_{2/2}-Einheit aufweisen, wobei R¹ gleich Alkylrest mit 2 bis 30 Kohlenstoffatomen, Arylrest oder Trimethylsiloxyrest und R² gleich Alkylrest mit 1 bis 30 Kohlenstoffatomen bedeutet. Des weiteren wird in GB-A-2 136 442 ein Antiklebrigmacher beschrieben, der ein Gemisch aus einer flüssigen und einer wachsartigen Organosiliciumverbindung enthält, wobei die wachsartige Komponente Stearoxydimethylsilan oder Distearoxydimethylsilan oder Dipolyoxyethylendimethylsilan darstellt. Solche wachsartigen Stearoxyverbindungen haben jedoch den Nachteil, daß sie aufgrund der reaktiven Si-O-C-Bindungen in kosmetischen Zubereitungen, insbesondere in wäßrigen, häufig unerwünschte Veränderungen hervorrufen, wie beispielsweise in der Konsistenz.

Gegenstand der Erfindung sind kosmetische Zusammensetzungen, die bei Raumtemperatur feste, wachsartige Organosiloxane der Formel

R¹R₂Si-O-SiR₂R¹ (I),

worin
- R: gleich oder verschieden sein kann und Alkylrest mit 1 bis 4 Kohlenstoffatomen und
- R¹: gleich oder verschieden sein kann und linearer Alkylrest mit mindestens 16 Kohlenstoffatomen bedeutet,
flüchtige Organosiloxane, ausgewählt aus der Gruppe, bestehend aus Hexamethyldisiloxan, Octamethylcyclotetrasiloxan, Decamethylcyclopentasiloxan sowie deren Gemische, und gegebenenfalls weitere Stoffe enthalten.

Unter Raumtemperatur soll im Sinne dieser Erfindung eine Temperatur von 20°C verstanden werden.

Beispiele für Rest R sind der Methyl- und Ethylrest sowie Propyl- und Butylreste, wobei der Methylrest besonders bevorzugt ist.

Beispiele für Rest R¹ sind der n-Hexadecylrest, n-Heptadecylrest, n-Octadecyl-, Eicosenyl- und Docosenylrest, wobei der n-Octadecylrest besonders bevorzugt ist.

Im Rahmen der vorliegenden Erfindung sollen bei den Organosiloxanen der Formel (I) die Reste R und R¹ nur so kombiniert werden können, daS ein bei Raumtemperatur festes, wachsartiges Organosiloxan resultiert.

Besonders bevorzugt handelt es sich bei dem Organosiloxan der Formel (I) um H₃₇C₁₈Si(CH₃)₂-O-Si(CH₃)₂C₁₈H₃₇.

Bevorzugt wird als flüchtiges Organosiloxan Octamethylcyclotetrasiloxan und Decamethylcyclopentasiloxan eingesetzt, wobei Octamethylcyclotetrasiloxan besonders bevorzugt ist.

Organosiloxan der Formel (I) sowie die genannten flüchtigen Organosiloxane sind handelsübliche Produkte sowie nach in der Silicon-Chemie gängigen Verfahren herstellbar.

In den erfindungsgemäßen Zusammensetzungen beträgt das Gewichtsverhältnis von festem, wachsartigen Organosiloxan zu flüchtigem Organosiloxan vorzugsweise 1 : 999 bis 2 : 3, besonders bevorzugt 1 : 99 bis 3 : 7, insbesondere 1 : 99 bis 15 : 85.

Bei den erfindungsgemäßen Zusammensetzungen kann es sich um beliebige Präparate zur Behandlung von Haut, Nägeln und Haaren handeln. Beispiele hierfür sind Emulsionen, wie Reinigungsemulsionen, flüssige Nährcremes, Körperlotionen, Sonnenschutzmittel und Bademilch, Cremes, wie etwa feste Cremes nach Art einer Nachtcreme, Hautnährcreme, Sonnenschutzcreme und dergleichen, sowie Stifte, wie Deodorantstift, Lippenstifte und Augenschminkstifte.

Vorzugsweise handelt es sich bei den erfindungsgemäßen Zusammensetzungen um Präparate zur Behandlung von Haut.

Je nach Einsatzgebiet können nun die erfindungsgemäßen Zusammensetzungen zusätzlich zu den wachsartigen Organosiloxanen der Formel (I) und den flüchtigen Organosiloxanen weitere, für die Bereitung von kosmetischen Präparaten übliche Stoffe enthalten.

Beispiele für gegebenenfalls eingesetzte Zusatzstoffe sind tierische, mineralische, pflanzliche oder synthetische Öle, Wachse oder Harze, Netzmittel, Fettalkohole, Emulgatoren, Sonnenfilter, organische Lösungsmittel, Verdickungsmittel, Farbstoffe, Pigmente, pH-Regler, Reduktionsmittel, Elektrolyte, Trübungszusätze, Konservierungsmittel, Antioxidantien, Permuttiermittel, Parfums, antiseborrhöische Mittel und Wirkstoffe, die der Behandlung, der Pflege und dem Schutz der Haut oder der Haare dienen können, sowie Wasser.

Art und Menge der gegebenenfalls eingesetzten Zusatzstoffe hängen vom jeweiligen Einsatzgebiet ab und sind im Bereich der Kosmetik weitreichend bekannt.

Bei den erfindungsgemäßen Zusammensetzungen handelt es sich vorzugsweise um solche, die festes, wachsartiges Organosiloxan der Formel (I) in Mengen von bevorzugt 0,1 bis 40 Gewichtsprozent, besonders bevorzugt 3 bis 10 Gewichtsprozent, insbesondere 3 bis 5 Gewichtsprozent, jeweils bezogen auf das Gesamtgewicht der erfindungsgemäßen Zusammensetzung, enthalten.

Zur Herstellung der erfindungsgemäßen Zusammensetzungen können wachsartiges Organosiloxan der Formel (I), flüchtiges Organosiloxan sowie die gegebenenfalls zugesetzten Stoffe auf beliebige Art und Weise miteinander vermischt werden.

Vorteilhafterweise wird jedoch das wachsartige Organosiloxan der Formel (I) zuerst in dem flüchtigen Organosiloxan gelöst und diese Lösung dann gegebenenfalls mit Zusatzstoffen vermischt.

Ein weiterer Gegenstand der Erfindung ist ein Verfahren zur Herstellung von kosmetischen Zusammensetzungen, dadurch gekennzeichnet, daß bei Raumtemperatur festes, wachsartiges Organosiloxan der Formel (I) in flüchtigem Organosiloxan, ausgewählt aus der Gruppe, bestehend aus, Hexamethyldisiloxan, Octamethylcyclotetrasiloxan, Decamethylcyclopentasiloxan sowie deren Gemische, gelöst wird und die resultierende Lösung gegebenenfalls mit weiteren Stoffe vermischt wird.

Das erfindungsgemäße Verfahren wird vorzugsweise bei 20 bis 70°C, besonders bevorzugt 20 bis 50°C und einem Druck von 900 bis 1100 hPa durchgeführt. Falls erwünscht, können zur besseren Einarbeitung die gegebenenfalls eingesetzten Zusatzstoffe abhängig von deren Beschaffenheit vor dem Mischen mit der Lösung aus Organosiloxan der Formel (I) und flüchtigem Organosiloxan für sich erwärmt und dann bei dieser Temperatur zugegeben werden.

Das erfindungsgemäße Verfahren hat den Vorteil, daß es sehr einfach in der Durchführung ist und eine homogene Wirkstoffverteilung ermöglicht.

Die erfindungsgemäßen Zusammensetzungen haben den Vorteil, daS das wachsartige Organosiloxan der Formel (I), welches ein wertvoller Wirkstoff im Zusammenhang mit Präparaten zur Körperpflege darstellt, darin homogen verteilt ist. Des weiteren haben die erfindungsgemäßen Zusammensetzungen den Vorteil, daß sie sehr leicht auftragbar und leicht verteilbar sind und nach einiger Zeit, insbesondere nach dem Abdampfen der flüchtigen Organosiloxane, einen gut haftenden und auch durch Feuchtigkeit oder Wasser nicht ohne weiteres entfernbaren Film ergeben, der ein trockenes und nicht klebriges Hautgefühl vermittelt. Vorteilhaft ist weiterhin die Tatsache, daß die erfindungsgemäßen Zusammensetzungen über einen langen Zeitraum stabil und hinsichtlich der Organosiloxane ihre Konsistenz nicht ändern.

In den nachstehend beschriebenen Beispielen beziehen sich alle Angaben von Teilen und Prozentsätzen, falls nicht anders angegeben, auf das Gewicht. Des weiteren beziehen sich alle Viskositätsangaben auf eine Temperatur von 25°C. Sofern nicht anders angegeben, wurden die nachstehenden Beispiele bei einem Druck der umgebenden Atmosphäre, also etwa 1000 hPa, und bei Raumtemperatur, also bei etwa 20°C, bzw. bei einer Temperatur, die sich beim Zusammengeben der Reaktanden bei Raumtemperatur ohne zusätzliche Heizung oder Kühlung einstellt, durchgeführt.

Im Folgenden soll unter der Bezeichnung "Stearylsiloxan" ein Disiloxan der Formel H₃₇C₁₈Si(CH₃)₂-O-Si(CH₃)₂C₁₈H₃₇ verstanden werden.

### Beispiel 1

1,00 Teile Paraffinöl (käuflich erhältlich unter der Bezeichnung "Paraffinöl dünnflüssig" bei Merck, Darmstadt), 1,00 Teile Cetylalkohol und 1,50 Teile Stearinsäure werden bei einer Temperatur von 85°C miteinander vermischt (Mischung A).

1,50 Teile Stearylsiloxan werden bei einer Temperatur von 50°C in 4,00 Teilen Decamethylcyclopentasiloxan gelöst (Mischung B).

0,80 Teile Triethanolamin, 3,00 Teile 1,2-Propandiol und 88,20 Teile Wasser werden bei einer Temperatur von 85°C miteinander vermischt (Mischung C).

Mischung B wird in Mischung A eingerührt und anschließend Mischung C zugegeben, wobei die Mischungen A, B und C jeweils die Temperatur ihrer Herstellung haben. In die so erhaltene Zubereitung werden noch 0,05 g Isothiazolinon (käuflich erhältlich unter der Bezeichnung "Kathon® CG" bei Rohm & Haas GmbH, Frankfurt), 0,1 g Duftstoff (käuflich erhältlich unter der Bezeichnung "Synambran®" bei der Wacker-Chemie GmbH, München) gegeben und auf Raumtemperatur abkühlen gelassen. Es resultiert eine dicke Lotion, die sehr schnell in die Haut einzieht und nicht fettet.
Die Lotion zeigt bei einer Lagerung bei 45°C und ca. 1000 hPa über einen Zeitraum von mehr als 10 Wochen keine Änderung der Konsistenz.

### Beispiel 2

5,50 Teile Candellilawachs und 3,00 Teile Stearinsäure werden bei einer Temperatur von 70°C miteinander vermischt (Mischung A).

6,70 Teile Stearylsiloxan werden bei einer Temperatur von 50°C in 18,30 Teilen Decamethylcyclopentasiloxan gelöst (Mischung B).

1,30 Teile Triethanolamin, 3,40 Teile 1,2-Propandiol und 44,80 Teile Wasser werden bei einer Temperatur von 70°C miteinander vermischt (Mischung C).

Mischung C wird in Mischung A eingerührt und anschließend Mischung B zugegeben, wobei die Mischungen A, B und C jeweils die Temperatur ihrer Herstellung haben. In die so erhaltene Zubereitung werden noch 14,00 g Titandioxid und 3,00 g Pigment (käuflich erhältlich unter der Bezeichnung "Sicomet-Braun® 70" bei BASF, Ludwigshafen) gegeben und auf Raumtemperatur abkühlen gelassen. Es resultiert eine cremig weiche Abdeckcreme mit sehr guter Deckkraft.
Die Abdeckcreme zeigt bei einer Lagerung bei 45°C und ca. 1000 hPa über einen Zeitraum von mehr als 10 Wochen keine Änderung der Konsistenz.

### Beispiel 3

1,00 Teile Stearylsiloxan werden bei einer Temperatur von 50°C in 25,00 Teilen Octamethylcyclotetrasiloxan gelöst (Mischung A).

69,00 Teile Paraffinöl (käuflich erhältlich unter der Bezeichnung "Paraffinöl dünnflüssig" bei Merck, Darmstadt) und 5,00 Teile Polypropylenglycol-15-Stearylether (käuflich erhältlich unter der Bezeichnung "Arlamol® E" bei der ICI, UK) werden bei einer Temperatur von 20°C miteinander vermischt (Mischung B).

Mischung B wird in Mischung A eingerührt, wobei die Mischungen A und B jeweils die Temperatur ihrer Herstellung haben. In die so erhaltene Zubereitung werden noch 0,05 g Isothiazolinon (käuflich erhältlich unter der Bezeichnung "Kathon® CG" bei Rohm & Haas GmbH, Frankfurt) und 0,2 g Duftstoff (käuflich erhältlich unter der Bezeichnung "Majantol®" bei der Wacker-Chemie GmbH, München) gegeben und auf Raumtemperatur abkühlen gelassen. Es resultiert ein farbloses, klares, dünnflüssiges Badeöl mit rückfettenden Eigenschaften. Das Badeöl zeigt bei einer Lagerung bei 45°C und ca. 1000 hPa über einen Zeitraum von mehr als 10 Wochen keine Änderung der Konsistenz.

## Patentansprüche

1. Kosmetische Zusammensetzungen, die bei Raumtemperatur feste, wachsartige Organosiloxane der Formel
R¹R₂Si-O-SiR₂R¹ (I),
worin
R gleich oder verschieden sein kann und Alkylrest mit 1 bis 4 Kohlenstoffatomen und
R¹ gleich oder verschieden sein kann und linearer Alkylrest mit mindestens 16 Kohlenstoffatomen bedeutet,
flüchtige Organosiloxane, ausgewählt aus der Gruppe, bestehend aus Hexamethyldisiloxan, Octamethylcyclotetrasiloxan, Decamethylcyclopentasiloxan sowie deren Gemische,
und gegebenenfalls weitere Stoffe
enthalten.

2. Kosmetische Zusammensetzung gemäß Anspruch 1, dadurch gekennzeichnet, daß es sich bei dem Organosiloxan der Formel (I) um H₃₇C₁₈Si(CH₃)₂-O-Si(CH₃)₂C₁₈H₃₇ handelt.

3. Kosmetische Zusammensetzung gemäß Anspruch 1 oder 2, dadurch gekennzeichnet, daß es sich bei dem flüchtigen Organosiloxan um Octamethylcyclotetrasiloxan handelt.

4. Kosmetische Zusammensetzung gemäß einem oder mehreren der Ansprüche 1 bis 3, dadurch gekennzeichnet, daß das Gewichtsverhältnis von festem, wachsartigen Organosiloxan der Formel (I) zu flüchtigem Organosiloxan 1 : 999 bis 2 : 3 beträgt.

5. Kosmetische Zusammensetzung gemäß einem oder mehreren der Ansprüche 1 bis 4, dadurch gekennzeichnet, daß sie festes, wachsartiges Organosiloxan der Formel (I) in Mengen von 0,1 bis 40 Gewichtsprozent, jeweils bezogen auf das Gesamtgewicht der erfindungsgemäßen Zusammensetzung, enthalten.

6. Verfahren zur Herstellung von kosmetischen Zusammensetzungen gemäß einem oder mehreren der Ansprüche 1 bis 5, dadurch gekennzeichnet, daß bei Raumtemperatur festes, wachsartiges Organosiloxan der Formel (I) in flüchtigem Organosiloxan, ausgewählt aus der Gruppe, bestehend aus, Hexamethyldisiloxan, Octamethylcyclotetrasiloxan, Decamethylcyclopentasiloxan sowie deren Gemische, gelöst wird und die resultierende Lösung gegebenenfalls mit weiteren Stoffe vermischt wird.

7. Verwendung der kosmetischen Zusammensetzungen gemäß einem oder mehreren der Ansprüche 1 bis 5 oder herstellbar nach Anspruch 6 zur Behandlung von Haut.

## Claims

1. Cosmetic composition which comprises a waxy organosiloxane, which is solid at room temperature, of the formula
R¹R₂Si-O-SiR₂R¹ (I),
in which
R can be identical or different and denotes an alkyl radical having 1 to 4 carbon atoms and
R¹ can be identical or different and denotes a linear alkyl radical having at least 16 carbon atoms,
a volatile organosiloxane chosen from the group comprising hexamethyldisiloxane, octamethylcyclotetrasiloxane, decamethylcyclopentasiloxane and mixtures thereof, and if appropriate other substances.

2. Cosmetic composition according to Claim 1, characterized in that the organosiloxane of the formula (I) is H₃₇C₁₈Si(CH₃)₂-O-Si(CH₃)₂C₁₈H₃₇.

3. Cosmetic composition according to Claim 1 or 2, characterized in that the volatile organosiloxane is octamethylcyclotetrasiloxane.

4. Cosmetic composition according to one or more of Claims 1 to 3, characterized in that the weight ratio of solid, waxy organosiloxane of the formula (I) to volatile organosiloxane is 1 : 999 to 2 : 3.

5. Cosmetic composition according to one or more of Claims 1 to 4, characterized in that it comprises a solid, waxy organosiloxane of the formula (I) in an amount of 0.1 to 40 per cent by weight, in each case based on the total weight of the composition according to the invention.

6. Process for the preparation of a cosmetic composition according to one or more of Claims 1 to 5, characterized in that a waxy organosiloxane, which is solid at room temperature, of the formula (I) is dissolved in a volatile organosiloxane chosen from the group comprising hexamethyldisiloxane, octamethylcyclotetrasiloxane, decamethylcyclopentasiloxane and mixtures thereof, and, if appropriate, the resulting solution is mixed with other substances.

7. Use of the cosmetic composition according to one or more of Claims 1 to 5 or which can be prepared according to Claim 6 for treatment of the skin.

## Revendications

1. Compositions cosmétiques qui contiennent des organosiloxanes cireux, solides à température ambiante, de formule
R¹R₂Si-O-SiR₂R¹ (I),
où
R peut être identique ou différent et représente un radical alcoyle avec 1 à 4 atomes de carbone, et
R¹ peut être identique ou différent et représente un radical alcoyle linéaire avec au moins 16 atomes de carbone,
des organosiloxanes volatils, choisis dans le groupe constitué de l'hexaméthyldisiloxane, l'octaméthylcyclotétrasiloxane, le décaméthylcyclopentasiloxane ainsi que leurs mélanges,
et facultativement, d'autres matières.

2. Composition cosmétique suivant la revendication 1, caractérisée en ce que l'organosiloxane de formule (I) consiste en H₃₇C₁₈Si(CH₃)₂-O-Si(CH₃)₂C₁₈H₃₇.

3. Composition cosmétique suivant la revendication 1 ou 2, caractérisée en ce que l'organosiloxane volatil consiste en l'octaméthylcyclotétrasiloxane.

4. Composition cosmétique suivant l'une ou plusieurs des revendications 1 à 3, caractérisée en ce que le rapport pondéral de l'organosiloxane cireux, solide, de formule (I) à l'organosiloxane volatil atteint 1:999 à 2:3.

5. Composition cosmétique suivant l'une ou plusieurs des revendications 1 à 4, caractérisée en ce qu'elle contient l'organosiloxane cireux, solide, de formule (I) en des quantités de 0,1 à 40% en poids, chaque fois sur base du poids total de la composition suivant l'invention.

6. Procédé de préparation de compositions cosmétiques suivant l'une ou plusieurs des revendications 1 à 5, caractérisé en ce que l'organosiloxane cireux, solide à température ambiante, de formule (I) est dissous dans l'organosiloxane volatil choisi dans le groupe constitué de l'hexaméthyldisiloxane, de l'octaméthylcyclotétrasiloxane, du décaméthylcyclopentasiloxane ainsi que leurs mélanges et que la solution résultante est facultativement mélangée à d'autres matières.

7. Utilisation des compositions cosmétiques suivant l'une ou plusieurs des revendications 1 à 5 ou préparées suivant la revendication 6, dans le traitement de la peau.
